# EUROPEAN PATENT APPLICATION

(11) **EP 2 492 258 A1**
(43) Date of publication of application: **29.08.2012**
(21) Application number: 10824703.2
(22) Date of filing: 08.07.2010
(51) Int. Cl.: C07C 67/347, C07C 69/757, C07B 61/00

(54) **METHOD FOR MANUFACTURING A TRICYCLODECANE MONO-METHANOL MONOCARBOXYLIC ACID DERIVATIVE**

(30) Priority: 22.10.2009 JP 2009243260
(71) Applicant: Hitachi Chemical Company, Ltd., Tokyo 163-0449 (JP); National Institute of Advanced Industrial Science and Technology, Tokyo 100-8921 (JP)
(72) Inventor: KAWAKAMI, Hiroyuki, Ichihara-shi Chiba 290-8567 (JP); TOMINAGA, Kenichi, Tsukuba-shi Ibaraki 305-8565 (JP); SATO, Kazuhiko, Tsukuba-shi Ibaraki 305-8565 (JP)
(74) Representative: HOFFMANN EITLE
(86) International application number: PCT/JP2010/061582
(87) International publication number: WO 2011/048851

(57) **Abstract**

Provided is a method for manufacturing a tricyclodecane mono-methanol monocarboxylic acid derivative that can be used as a raw material for high heat-resistant alicyclic polyesters and the like.

The provided method for manufacturing a tricyclodecane mono-methanol monocarboxylic acid derivative is characterized in that a tricyclodecane mono-methanol monocarboxylic acid derivative represented by the following formula (III) is obtained by reacting the dicyclopentadiene represented by the following formula (I) with a formic acid compound in the presence of a catalytic system containing a ruthenium compound, a cobalt compound, and a halide salt, to give a tricyclodecene monocarboxylic acid derivative represented by the formula (II), and then hydroformylating the tricyclodecene monocarboxylic acid derivative. 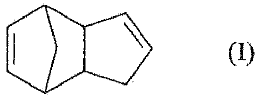 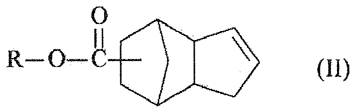 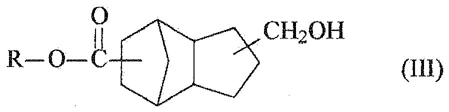 (in the formulae (II) and (III), R represents a hydrogen, an alkyl group having 1 to 5 carbon atoms, a vinyl group, or a benzyl group.)

## Description

### TECHNICAL FIELD

The present invention relates to a method for manufacturing a raw material for high heat-resistant alicyclic polyesters and the like.

### BACKGROUND ART

Various polyester resins can be molded into a film, a sheet, a deformed material, a fiber, a tube, a container or the like by various kinds of molding methods. Thus, they are used in a broad field. The most commonly used polyester is an aromatic polyester made from aromatic dicarboxylic acids such as terephthalic acid or isophthalic acid as a raw material and it has excellent heat resistance and toughness because it contains an aromatic group.

Recently, a semiconductor laser source with a low wavelength range is used, and blue laser, UV laser, and the like are used as a light source for light signal. Accordingly, it is required that an optical material or a polymer material used for electronic parts and the like has transparency. However, since the aromatic polyester has poor UV resistance and poor light transmission, it cannot be applied to such fields.

Polyesters having an alicyclic structure have excellent heat resistance, transparency, and water resistance, and thus some of them are used in the fields in which the transparency is required. As a method for manufacturing alicyclic polyesters, various methods of using saturated cyclic aliphatic primary diols such as 1,4-cyclohexane dimethanol are suggested (Patent Document 1). Since an alkylene group is inserted between a hydroxyl group and a saturated cyclic aliphatic group in the saturated cyclic aliphatic primary diols, the alicyclic polyester resins obtained therefrom have aliphatic properties and the resins having a cyclohexane ring have low heat resistance. Thus, sufficient properties are not provided for the uses described above.

For the purpose of enhancing heat resistance of alicyclic polyesters, alicyclic polyesters consisting of alicyclic diol and dicarboxylic acid component containing 4,4'-bicyclohexyldicarboxylic acid as a main component are suggested (Patent Document 2). However, the heat resistance is still insufficient.

Meanwhile, (meth)acrylic acid esters having a tricyclodecane skeleton show excellent curability, water resistance, plasticity, and alkali resistance, and therefore various studies have been done regarding them (Patent Documents 3 to 5). However, polyesters having a tricyclodecane skeleton are not known.
In this connection, a raw material for alicyclic polyesters having excellent heat resistance has been waited for.

### CITATION LIST

### PATENT DOCUMENTS

Patent Document 1: Japanese Patent Application National Publication No. 2007-517926
Patent Document 2: Japanese Patent Application Laid-Open (JP-A) No. 2006-111794
Patent Document 3: JP-A No. 8-311130
Patent Document 4: JP-A No. 2002-265886
Patent Document 5: JP-A No. 2006-315960

### SUMMARY OF THE INVENTION

One embodiment of the invention is (1): a method for manufacturing a tricyclodecane mono-methanol monocarboxylic acid derivative, comprising the steps of reacting a dicyclopentadiene represented by formula (I) with a formic acid compound in the presence of a catalytic system containing a ruthenium compound, a cobalt compound, and a halide salt, to give a tricyclodecene monocarboxylic acid derivative represented by formula (II), and hydroformylating the tricyclodecene monocarboxylic acid derivative to give a tricyclodecane mono-methanol monocarboxylic acid derivative represented by formula (III). 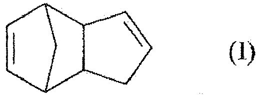 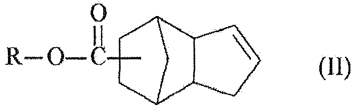 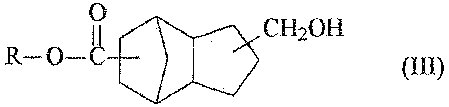 (in the formulae (II) and (III), R represents a hydrogen, an alkyl group having 1 to 5 carbon atoms, a vinyl group, or a benzyl group.)

Further, one embodiment of the invention is (2): the method for manufacturing a tricyclodecane mono-methanol monocarboxylic acid derivative described in above (1), wherein the ruthenium compound is a ruthenium complex having both a carbonyl ligand and a halogen ligand in the molecule.
Further, one embodiment of the invention is (3): the method for manufacturing a tricyclodecane mono-methanol monocarboxylic acid derivative described in above (1) or (2), wherein the halide salt is a quaternary ammonium salt.

Further, one embodiment of the invention is (4): the method for manufacturing a tricyclodecane mono-methanol monocarboxylic acid derivative described in any one of above (1) to (3), wherein the reaction between the dicyclopentadiene and formic acid compound is performed in the presence of a basic compound.

Further, one embodiment of the invention is (5): the method for manufacturing a tricyclodecane mono-methanol monocarboxylic acid derivative described in above (4), wherein the basic compound is a tertiary amine.

Further, one embodiment of the invention is (6): the method for manufacturing a tricyclodecane mono-methanol monocarboxylic acid derivative described in any one of above (1) to (5), wherein the reaction between the dicyclopentadiene and formic acid compound is performed in the presence of a phenol compound.

Further, one embodiment of the invention is (7): the method for manufacturing a tricyclodecane mono-methanol monocarboxylic acid derivative described in any one of above (1) to (6), wherein the reaction between the dicyclopentadiene and formic acid compound is performed in the presence of an organic halide.

Further, one embodiment of the invention is (8): the method for manufacturing a tricyclodecane mono-methanol monocarboxylic acid derivative described in any one of above (1) to (7), wherein the hydroformylation of the tricyclodecene monocarboxylic acid derivative is performed in the presence of a catalytic system containing a ruthenium compound.

Further, one embodiment of the invention is (9): the method for manufacturing a tricyclodecane mono-methanol monocarboxylic acid derivative described in any one of above (1) to (8), wherein a halide salt is used in combination in the catalytic system for the hydroformylation of the tricyclodecene monocarboxylic acid derivative.

Further, one embodiment of the invention is (10): the method for manufacturing a tricyclodecane mono-methanol monocarboxylic acid derivative described in any one of above (1) to (9), wherein a cobalt compound is used in combination in the catalytic system for the hydroformylation of the tricyclodecene monocarboxylic acid derivative.

Further, one embodiment of the invention is (11): the method for manufacturing a tricyclodecane mono-methanol monocarboxylic acid derivative described in any one of above (1) to (10), wherein an acid is used in combination in the catalytic system for the hydroformylation of the tricyclodecene monocarboxylic acid derivative.

Further, one embodiment of the invention is (12): the method for manufacturing a tricyclodecane mono-methanol monocarboxylic acid derivative described in above (11), wherein the acid is Broensted acid.

Further, one embodiment of the invention is (13): the method for manufacturing a tricyclodecane mono-methanol monocarboxylic acid derivative described in above (12), wherein the Broensted acid is an acid containing phosphorus.

Further, one embodiment of the invention is (14): the method for manufacturing a tricyclodecane mono-methanol monocarboxylic acid derivative described in any one of above (1) to (13), wherein the hydroformylation of the tricyclodecene monocarboxylic acid derivative is performed in the presence of a phenol compound.

Still further, one embodiment of the invention is (15): the method for manufacturing a tricyclodecane mono-methanol monocarboxylic acid derivative described in any one of above (1) to (14), wherein the hydroformylation of the tricyclodecene monocarboxylic acid derivative is performed at a reaction temperature of 100 to 200°C and pressure of 1 to 20 MPa.
The subject matter of the present invention is described in Japanese Patent Application No. 2009-243260 which has been filed on October 22, 2009, and the entire content thereof is incorporated herein by reference.

According to one embodiment of the invention, a method for manufacturing a tricyclodecane mono-methanol monocarboxylic acid derivative that can be used as a raw material for high heat-resistant alicyclic polyesters and the like is provided.

### BRIEF DESCRIPTION OF THE DRAWINGS

FIG. 1 is a ¹H-NMR spectrum of the methyl tricyclodecane mono-methanol monocarboxylate which is obtained from Example 11.
FIG. 2 is a FT-IR spectrum of the methyl tricyclodecane mono-methanol monocarboxylate which is obtained from Example 11.

### EMBODIMENTS FOR CARRYING OUT THE INVENTION

Herein below, the invention is explained more specifically. One embodiment of the invention relates to a method of manufacturing a tricyclodecane mono-methanol monocarboxylic acid derivative that is represented by the following formula (III).

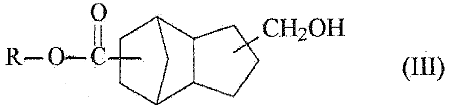

The tricyclodecane mono-methanol monocarboxylic acid derivative obtained by the manufacturing method of the invention is tricyclodecane mono-methanol monocarboxylic acid represented by the formula (III) or an ester thereof. In the formula (III), R represents a hydrogen; an alkyl group having 1 to 5 carbon atoms such as a methyl group, an ethyl group, a propyl group, an isopropyl group, a butyl group, a tert-butyl group, and a pentyl group; a vinyl group; or a benzyl group. Of these, from the viewpoint of high reactivity and low production cost, an alkyl group having 1 to 5 carbon atoms is preferable. Methyl group is particularly preferable.
In the formula (III), a binding site of two substituent groups, i.e., -C(O)OR and - CH₂OH, is not particularly limited. However, it is preferable that -C(O)OR binds to position 8 or position 9 of the tricyclodecane group and -CH₂OH binds to position 3 or position 4 of the tricyclodecane group.
Examples of the tricyclodecane mono-methanol monocarboxylic acid and derivative thereof include 4-hydroxymethyl-8-carboxy-tricyclo[5.2.1.0^{2.6}]decane, 3-hydroxymethyl-8-carboxy-tricyclo[5.2.1.0^{2,6}]decane, 3-hydroxymethyl-9-carboxy-tricyclo[5.2.1.0^{2,6}]decane, 4-hydroxymethyl-8-methoxycarbonyl-tricyclo[5.2.1.0^{2,6}]decane, 3-hydroxymethyl-8-methoxycarbonyl-tricyclo[5.2.1.0^{2,6}]decane, 3-hydroxymethyl-9-methoxycarbonyl-tricyclo[5.2.1.0^{2,6}]decane, 4-hydroxymethyl-8-butoxycarbonyl-tricyclo[5.2.1.0^{2,6}]decane, 3-hydroxymethyl-8-butoxycarbonyl-tricyclo[5.2.1,0²,⁶ ]decane, 3-hydroxymethyl-9-butoxycarbonyl-tricyclo[5.2.1.0^{2,6}]decane, 4-hydroxymethyl-8-pentoxycarbonyl-tricyclo[5.2.1.0^{2,6}]decane, 3-hydroxymethyl-8-pentoxycarbonyl-tricyclo[5.2.1.0^{2,6}]decane, 3-hydroxymethyl-9-pentoxycarbonyl-tricyclo[5.2.1.0^{2,6}]decane, 4-hydroxymethyl-8-vinyloxycarbonyl-tricyclo[5.2.1.0^{2,6}]decane, 3-hydroxymethyl-8-vinyloxycarbonyl-tricyclo[5.2.1.0^{2,6}]decane, 3-hydroxymethyl-9-vinyloxycarbonyl-tricyclo[5.2.1.0^{2,6}]decane, 4-hydroxymethyl-8-benzyloxycarbonyl-tricyclo[5.2.1.0^{2,6}]decane, 3-hydroxymethyl-8-benzyloxycarbonyl-tricyclo[5.2.1.0^{2,6}]decane, and 3-hydroxymethyl-9-benzyloxycarbonyl-tricyclo[5.2.1.0^{2,6}]decane.

The method for manufacturing the tricyclodecane mono-methanol monocarboxylic acid derivative according to the invention is characterized in that the tricyclodecane mono-methanol monocarboxylic acid derivative represented by the formula (III) is obtained by reacting the dicyclopentadiene represented by the following formula (I) with a formic acid compound in the presence of a catalytic system containing a ruthenium compound, a cobalt compound, and a halide salt, to give a tricyclodecene monocarboxylic acid derivative represented by the following formula (II), and then hydroformylating the tricyclodecene monocarboxylic acid derivative. 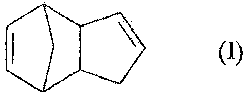 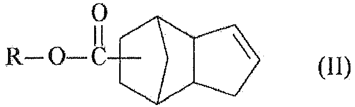 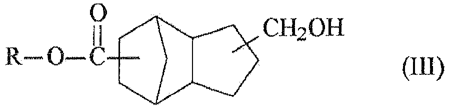 (in formulae (II) and (III), R represents a hydrogen, an alkyl group having 1 to 5 carbon atoms, a vinyl group, or a benzyl group).

According to the manufacturing method of the invention, the dicyclopentadiene represented by the following formula (I) is first reacted with a formic acid compound (HCOOR) for adding -C(O)OR in the presence of a catalytic system containing a ruthenium compound, a cobalt compound, and a halide salt to yield a tricyclodecene monocarboxylic acid derivative represented by the following formula (II). 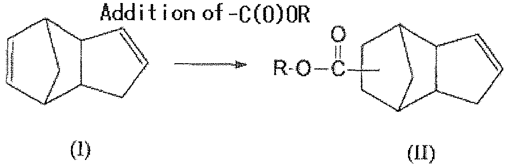 As for the formic acid compound (HCOOR), when formic acid wherein R is hydrogen is used, a hydrocarboxylation reaction occurs so that a carboxyl group can be added to dicyclopentadiene. When a formic acid ester wherein R is an alkyl group having 1 to 5 carbon atoms, a vinyl group, or a benzyl group is used, a hydroesterification occurs so that an ester group can be added to dicyclopentadiene.
As a formic acid compound, a formic acid compound (HCOOR) corresponding to - C(O)OR of the tricyclodecane mono-methanol monocarboxylic acid derivative is used. Examples of the formic acid compound include formic acid, methyl formate, ethyl formate, propyl formate, isopropyl formate, butyl formate, isobutyl formate, amyl formate, isoamyl formate, vinyl formate, benzyl formate, and the like. Of these, from the viewpoint of production cost and reactivity, methyl formate is preferable.

The reaction between dicyclopentadiene represented by the formula (I) and the formic acid compound is performed in the presence of a catalytic system containing a ruthenium compound, a cobalt compound, and a halide salt. The ruthenium compound that can be used in the invention is not specifically limited, as long as it contains ruthenium. Specific examples of the ruthenium compound that can be preferably used in the invention include a ruthenium compound containing both a carbonyl ligand and a halogen ligand in the molecule such as [Ru(CO)₃Cl₂]₂, [RuCb(CO)₂]ₙ (n is an unspecified natural number), [Ru(CO)₃Cl₃]⁻, [Ru₃(CO)₁₁Cl]⁻, and [Ru₄(CO)₁₃Cl]⁻. Of these, from the viewpoint of increasing reaction ratio, [Ru(CO)₃Cl₂]₂ and [RuCl₂(CO)₂]ₙ are preferable.

The ruthenium compound containing both ligands can be produced by using a precursor compound such as RuCl₃, Ru₃(CO)₁₂, RuCl₂(C₈H₁₂), Ru(CO)₃(C₈H₈), Ru(CO)₃(C₈H₁₂), and Ru(C₈H₁₀)(C₈H₁₂).

The use amount of the ruthenium compound is preferably 1/10000 to 1 equivalent, and more preferably 1/1000 to 1/50 equivalent compared to 1 equivalent of the dicyclopentadiene as a raw material. From the viewpoint of reaction rate, 1/10000 equivalent or more is preferable. Further, from the viewpoint of production cost, 1 equivalent or less is preferable.

The cobalt compound that can be used in the invention is not specifically limited, as long as it is a compound containing cobalt. Specific examples of the cobalt compound preferably include a cobalt compound having a carbonyl ligand such as Co₂(CO)₈, Co(CO)₄, and Co₄(CO)₁₂, a cobalt compound having a carboxylic acid compound as a ligand such as cobalt acetate, cobalt propionate, cobalt benzoate, and cobalt citrate, and cobalt phosphate. Of these, from the viewpoint of increasing reactivity, Co₂(CO)₈, cobalt acetate, and cobalt citrate are more preferable.

The use amount of the cobalt compound is preferably 1/100 to 10 equivalents, and more preferably 1/10 to 5 equivalents compared to 1 equivalent of the ruthenium compound, from the viewpoint of production amount of the tricyclodecene monocarboxylic acid derivative that is represented by the formula (II).

The halide salt that can be used in the invention is not specifically limited, as long as it is a compound consisting of a halogen ion and a cation. Examples of the halogen ion include a chloride ion, a bromide ion, and an iodide ion. The cation may be any one of an inorganic ion and an organic ion. Further, the halide salt may contain one or more halogen ions in the molecule.

The inorganic ion which constitutes the halide salt may be any one metal ion selected from an alkali metal and an alkali earth metal. Specific examples thereof include lithium, sodium, potassium, rubidium, cesium, calcium, and strontium.

Further, the organic ion may be an organic group having a valency of 1 or more which is derived from an organic compound. Examples thereof include ammonium, phosphonium, pyrrolidinium, pyridium, imidazolium, and iminium. The hydrogen atoms contained in these ions may be substituted with a hydrocarbon group such as alkyl and aryl. Specific examples of the organic ion preferably include, although not specifically limited, tetramethyl ammonium, tetraethyl ammonium, tetrapropyl ammonium, tetrabutyl ammonium, tetrapentyl ammonium, tetrahexyl ammonium, tetraheptyl ammonium, tetraoctyl ammonium, trioctyl methyl ammonium, benzyl trimethyl ammonium, benzyl triethyl ammonium, benzyl tributyl ammonium, tetramethyl phosphonium, tetraethyl phosphonium, tetraphenyl phosphonium, benzyl triphenyl phosphonium, butyl methyl pyrrolidinium, octyl methyl pyrrolidinium, and bis(triphenylphosphine)iminium. Of these, from the viewpoint of increasing reaction ratio, butyl methyl pyrrolidinium, bis(triphenylphosphine)iminium, trioctyl methyl ammonium, and the like are more preferable.

It is not necessary that the halide salt used in the invention is a solid salt. An ionic liquid containing a halide ion which becomes a liquid at near room temperature or the temperature region of 100°C or lower may be also used. Examples of the cation that is employed in the ionic liquid include an organic ion such as 1-ethyl-3-methylimidazolium, 1-propyl-3-methylimidazolium, 1-butyl-3-methylimidazolium, 1-pentyl-3-methylimidazolium, 1-hexyl-3-methylimidazolium, 1-heptyl-3-methylimidazolium, 1-octyl-3-methylimidazolium, 1-decyl-3-methylimidazolium, 1-dodecyl-3-methylimidazolium, 1-tetradecyl-3-methylimidazolium, 1-hexadecyl-3-methylimidazolium, 1-octadecyl-3-methylimidazolium, 1-ethyl-2,3-dimethylimidazolium, 1-butyl-2,3-dimethylimidazolium, 1-hexyl-2,3-dimethylimidazolium, 1-ethylpyridinium, 1-butylpyridinium, 1-hexylpyridinium, 8-methyl-1,8-diazabicyclo[5.4.0]-7-undecene, 8-ethyl-1,8-diazabicyclo[5.4.0]-7-undecene, 8-propyl-1,8-diazabicyclo[5.4.0]-7-undecene, 8-butyl-1,8-diazabicyclo[5.4.0]-7-undecene, 8-pentyl-1,8-diazabicyclo[5.4.0]-7-undecene, 8-hexyl-1,8-diazabicyclo[5.4.0]-7-undecene, 8-heptyl-1,8-diazahicyclo[5.4.0]-7-undecene, and 8-octyl-1,8-diazabicyclo[5.4.0]-7-undecene. According to the invention, the halide salt described in the above may be used either singly or in combination of two or more.

Among the halide salts described above, preferred halide salt includes a chloride salt, a bromide salt, and an iodide salt, wherein the cation is an organic ion. Although not specifically limited, specific examples of the halide salt preferably include butyl methyl pyrrolidinium chloride, bis(triphenylphosphine)iminium iodide, trioctyl methyl ammonium chloride, and the like.

The addition amount of the halide salt is preferably 1 to 1000 equivalents and more preferably 2 to 50 equivalents compared to 1 equivalent of the ruthenium compound. By adding the salt in an amount of 1 equivalent or more, the reaction rate can be effectively increased. Meanwhile, from the viewpoint of the effect of promoting the reaction, it is preferably 1000 equivalents or less.

Regarding the reaction between dicyclopentadiene and the formic acid compound according to the invention, the effect of promoting the reaction by the catalytic system can be further increased by adding, if necessary, at least one kind selected from a basic compound, a phenol compound, and an organic halogen compound to the specific catalytic system containing a ruthenium compound, a cobalt compound, and a halide salt.

The basic compound used in the invention may be either an inorganic compound or an organic compound. Specific examples of the inorganic basic compound include a carbonate salt, a hydrogen carbonate salt, a hydroxide salt, an alkoxide, and the like of an alkali metal and an alkali earth metal. Specific examples of the organic basic compound include a primary amine compound, a secondary amine compound, a tertiary amine compound, a pyridine compound, an imidazole compound, and a quinoline compound. Among the basic compounds described above, from the viewpoint of an effect of promoting the reaction, the tertiary amine compound is preferable. Specific examples of the tertiary amine compound that can be preferably used in the invention include trialkylamine, N-alkylpyrrolidine, quinuclidine, triethylene diamine, and the like.

The addition amount of the basic compound is, although not specifically limited, preferably 1 to 1000 equivalents and more preferably 2 to 200 equivalents compared to 1 equivalent of the ruthenium compound. By adding the salt in an amount of 1 equivalent or more, the effect of promoting the reaction tends to be more significant. Further, from the viewpoint of the effect of promoting the reaction, it is preferably 1000 equivalents or less.

The phenol compound that can be used in the invention is not specifically limited. Specific examples of the usable phenol compound include phenol, cresol, alkylphenol, methoxyphenol, phenoxyphenol, chlorophenol, trifluoromethylphenol, hydroquinone, catechol, and the like.

The addition amount of the phenol compound is, although not specifically limited, preferably 1 to 1000 equivalents and more preferably 2 to 200 equivalents compared to 1 equivalent of the ruthenium compound. By adding the phenol compound in an amount of 1 equivalent or more, the effect of promoting the reaction tends to be more significant. Further, from the viewpoint of the reaction efficiency, it is preferably 1000 equivalents or less.

The organic halogen compound that can be used in the invention is not specifically limited. Specific examples of the usable organic halogen compound include methane monohalide, methane dihalide, ethane dihalide, methane trihalide, methane tetrahalide, benzene halide, and the like.

The addition amount of the organic halogen compound is, although not specifically limited, preferably 1 to 1000 equivalents and more preferably 2 to 200 equivalents compared to 1 equivalent of the ruthenium compound. By adding the organic halogen compound in an amount of 1 equivalent or more, the effect of promoting the reaction tends to be more significant. Further, from the viewpoint of the reaction efficiency, it is preferably 1000 equivalents or less.

The reaction between dicyclopentadiene and the formic acid compound according to the invention can be performed without specifically using a solvent. However, if necessary, a solvent may be used. The solvent that can be used is not specifically limited, as long as it can dissolve the compounds that are used as a raw material. Specific examples of the solvent that can be preferably used include n-pentane, n-hexane, n-heptane, cyclohexane, benzene, toluene, o-xylene, p-xylene, m-xylene, ethylbenzene, cumen, tetrahydrofuran, N-methyl pyrrolidone, dimethyl formamide, dimethyl acetamide, dimethyl imidazolidinone, ethylene glycol dimethyl ether, diethylene glycol dimethyl ether, triethylene glycol dimethyl ether, and the like.

The reaction between dicyclopentadiene and the formic acid compound according to the invention is preferably carried out in the temperature range of 80°C to 200°C. More preferably, it is carried out at a temperature of 100°C to 160°C. When the reaction is carried out at a temperature of 80°C or higher, the reaction rate increases so that the efficient reaction can be conducted easily. Further, by controlling the reaction temperature at 200°C or lower, decomposition of the formic acid compound that is used as a raw material can be prevented. When the formic acid compound is decomposed, addition of a carboxylic acid or an ester group to dicyclopentadiene is not achieved, and therefore excessively high reaction temperature is not desirable. When the reaction temperature is above the boiling point of any one of the dicyclopentadiene and formic acid compound to be used as a raw material, it is necessary to carry out the reaction within a pressure resistant vessel. Completion of the reaction can be confirmed by a well-known analytical method such as gas chromatography and NMR. Further, the reaction is preferably carried out in the presence of an inert gas such as nitrogen and argon.

The tricyclodecene monocarboxylic acid derivative represented by the formula (II), which is obtained by the reaction between dicyclopentadiene represented by the formula (I) and the formic acid compound, is a dicyclopentadiene compound represented y the formula (I) in which -C(O)OR is added, and it has a structure of having -C(O)OR at position 8 or position 9 of the skeleton of tricyclo[5.2.1.0^{2,6}]dec-3-ene.

According to the manufacturing method of the invention, the tricyclodecene monocarboxylic acid derivative represented by the following formula (II) obtained by the method described above is subsequently hydroformylated to give the tricyclodecane mono-methanol monocarboxylic acid derivative represented by the following formula (III).

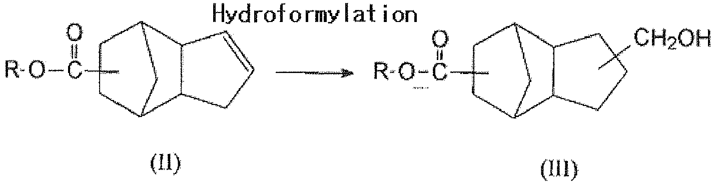

The hydroformylation reaction of the tricyclodecene monocarboxylic acid derivative represented by the formula (II) can be carried out by a hydroformylation method conventionally known in the field. As a conventional hydroformylation method, a method of adding an aldehyde by reacting carbon monoxide and hydrogen using a transition metal complex catalyst such as cobalt, ruthenium, and rhodium followed by hydrogenation, or a method of directly adding an alcohol by reacting carbon monoxide with hydrogen is disclosed in Catalyst Course, Vol. 7, Catalysis Society of Japan, Kodansha Ltd. (1985).

Meanwhile, as highly toxic carbon monoxide is used for conventional hydroformylation method, from the viewpoint of workability, safety, reactivity, and the like, the hydroformylation method using carbon dioxide and hydrogen as described below is preferable. In such case, carbon dioxide and hydrogen may be supplied in the form of a mixture gas, or they may be supplied separately. The mixture gas is a mixture gas (raw material gas) which contains carbon dioxide and hydrogen as a main component. Content of the carbon dioxide is preferably 10 to 95 vol%, and more preferably 50 to 80 vol%. Content of the hydrogen is preferably 5 to 90 vol%, and more preferably 20 to 50 vol%. When the content of the hydrogen is less than 90 vol%, hydrogenation of the raw material may not be easily obtained. On the other hand, when it is more than 5%, the reaction rate is high. It is not necessary that carbon monoxide is included in a raw material gas. However, it is not a problem even if it is included in a raw material gas.

The catalytic system for the hydroformylation preferably contains a ruthenium compound. The ruthenium compound that can be used is not specifically limited, as long as it is a compound containing ruthenium. Specific examples of the ruthenium compound include a ruthenium compound containing both a carbonyl ligand and a halogen ligand in the molecule such as [Ru(CO)₃Cl₂]₂, [RuCl₂(CO)₂]ₙ (n is an unspecified natural number), [Ru(CO)₃Cl₃]⁻, [Ru₃(CO)₁₁Cl]⁻, and [Ru₄(CO)₁₃Cl]⁻, and the like. Of these, from the viewpoint of increasing reaction ratio, [Ru(CO)₃Cl₂]₂ and [RuCl₂(CO)₂]ₙ are preferable.

The ruthenium compound containing both ligands can be produced by using a precursor compound such as RuCl₃, Ru₃(CO)₁₂, RuCl₂(C₈H₁₂), Ru(CO)₃(C₈H₈), Ru(CO)₃(C₈H₁₂), and Ru(C₈H₁₀)(C₈H₁₂).

The use amount of the ruthenium compound is preferably 1/10000 to 1 equivalent, and more preferably 1/1000 to 1/50 equivalent compared to 1 equivalent of the tricyclodecene monocarboxylic acid derivative represented by the formula (II). From the viewpoint of production cost, it is preferable to use less amount of the ruthenium compound. However, from the viewpoint of reaction rate, it is preferably 1/10000 equivalent or more. Further, from the viewpoint of the production cost, it is preferably 1 equivalent or less.

For the hydroformylation of the tricyclodecene monocarboxylic acid derivative represented by the formula (II), by adding, if necessary, at least one selected from a cobalt compound, a halide salt, a phenol compound, and an acid to the reaction system containing the ruthenium compound, the effect of promoting reaction by the reaction system can be enhanced more.

The cobalt compound that can be used as a catalyst for the hydroformylation is not specifically limited, as long as it is a compound containing cobalt. Specific examples of the cobalt compound include a cobalt compound containing a carbonyl ligand such as Co₂(CO)₈, HCo(CO)₄, and Co₄(CO)₁₂, a cobalt compound containing a carboxylic acid compound as a ligand such as cobalt acetate, cobalt propionate, cobalt benzoate, and cobalt citrate, cobalt phosphate, and the like. Of these, from the viewpoint of enhancing reaction ratio, Co₂(CO)₈, cobalt acetate, and cobalt citrate are more preferable.

The use amount of the cobalt compound is preferably 1/100 to 10 equivalents, and more preferably 1/10 to 5 equivalents compared to 1 equivalent of the ruthenium compound, from the viewpoint of the production amount of the tricyclodecane mono-methanol monocarboxylic acid derivative.

The halide salt that can be used in the invention is not specifically limited, as long as it is a compound consisting of a halogen ion and a cation. Examples of the halogen ion include a chloride ion, a bromide ion, an iodide ion, and the like. The cation may be any one of an inorganic ion and an organic ion. In addition, the halide salt may contain one or more halogen ions in the molecule.

The inorganic ion which constitutes the halide salt may be one kind of metal ion selected from an alkali metal and an alkali earth metal. Specific examples thereof include lithium, sodium, potassium, rubidium, cesium, calcium, and strontium.

Further, the organic ion may be an organic group having a valency of 1 or more which is derived from an organic compound. Examples thereof include ammonium, phosphonium, pyrrolidinium, pyridium, imidazolium, and iminium. The hydrogen atoms contained in these ions may be substituted with a hydrocarbon group such as alkyl and aryl. Specific examples of the organic ion preferably include, although not specifically limited, tetramethyl ammonium, tetraethyl ammonium, tetrapropyl ammonium, tetrabutyl ammonium, tetrapentyl ammonium, tetrahexyl ammonium, tetraheptyl ammonium, tetraoctyl ammonium, trioctyl methyl ammonium, benzyl trimethyl ammonium, benzyl triethyl ammonium, benzyl tributyl ammonium, hexadecyl trimethyl ammonium, tetramethyl phosphonium, tetraethyl phosphonium, tetraphenyl phosphonium, benzyl triphenyl phosphonium, butyl methyl pyrrolidinium, octyl methyl pyrrolidinium, and bis(triphenylphosphine)iminium. Of these, from the viewpoint of increasing reaction ratio, a quaternary ammonium salt such as hexadecyl trimethyl ammonium chloride and hexadecyl trimethyl ammonium bromide is more preferable.

It is not necessary that the halide salt used in the invention is a solid salt. An ionic liquid containing a halide ion which becomes a liquid at near room temperature or the temperature region of 100°C or lower may be also used. Examples of the cation that is employed in the ionic liquid include an organic ion such as I-ethyl-3-methylimidazolium, 1-propyl-3-methylimidazolium, 1-butyl-3-methylimidazolium, I-pentyl-3-methylimidazolium, 1-hexyl-3-methylimidazolium, 1-heptyl-3-methylimidazolium, 1-octyl-3-methylimidazolium, 1-decyl-3-methylimidazolium, 1-dodecyl-3-methylimidazolium, 1-tetradecyl-3-methylimidazolium, 1-hexadecyl-3-methylimidazolium, 1-octadecyl-3-methylimidazolium, 1-ethyl-2,3-dimethylimidazolium, 1-butyl-2,3-dimethylimidazolium, 1-hexyl-2,3-dimethylimidazolium, 1-ethylpyridinium, 1-butylpyridinium, 1-hexylpyridinium, 8-methyl-1,8-diazabicyclo[5.4.0]-7-undecene, 8-ethyl-1,8-diazabicyclo[5.4.0]-7-undecene, 8-propyl-1,8-diazabicyclo[5.4.0]-7-undecene, 8-butyl-1,8-diazabicyclo[5.4.0]-7-undecene, 8-pentyl-1,8-diazabicyclo[5.4.0]-7-undecene, 8-hexyl-1,8-diazabicyclo[5.4.0]-7-undecene, 8-heptyl-1,8-diazabicyclo[5.4.0]-7-undecene, and 8-octyl-1,8-diazabicyclo[5.4.0]-7-undecene. According to the invention, the halide salt described in the above may be used either singly or in combination of two or more.

Among the halide salts described above, preferred halide salt includes a chloride salt, a bromide salt, and an iodide salt, wherein the cation is an organic ion. Although not specifically limited, specific examples of the halide salt preferably include hexadecyl trimethyl ammonium chloride, hexadecyl trimethyl ammonium bromide, and the like.

The addition amount of the halide salt is preferably 1 to 1000 equivalents and more preferably 2 to 50 equivalents compared to 1 equivalent of the ruthenium compound. By adding the salt in an amount of 1 equivalent or more, the reaction rate can be effectively increased. Meanwhile, from the viewpoint of the effect of promoting the reaction, it is preferably 1000 equivalents or less.

The phenol compound that can be used in the invention is not specifically limited. Examples of the usable phenol compound include phenol, cresol, alkylphenol, methoxyphenol, phenoxyphenol, chlorophenol, trifluoromethylphenol, hydroquinone, catechol, and the like.

The addition amount of the phenol compound is, although not specifically limited, preferably 1 to 1000 equivalents and more preferably 2 to 200 equivalents compared to 1 equivalent of the ruthenium compound. By adding the phenol compound in an amount of 1 equivalent or more, the effect of promoting the reaction tends to be more significant. Further, from the viewpoint of the effect of promoting the reaction, it is preferably 1000 equivalents or less.

As an acid that can be used for the invention, all acids falling within the definition of Lewis acid can be used. According to the definition, when a certain substance "A" receives an electron pair from other substance "B", "A" is defined as an acid and "B" is defined as a base. All acids corresponding to electron pair-receiving "A" can be used.

As for the acid described above, an acid in which A is a proton donor, i.e., Broensted acid, is preferable. Examples of the Broensted acid include hydrochloric acid, sulfuric acid, nitric acid, phosphoric acid, methyl phosphoric acid, alkyl phosphoric acid, phenyl phosphoric acid, diphenyl phosphite, phenyl phosphonic acid, 4-methoxyphenyl phosphonic acid, diethyl 4-methoxyphenyl phosphonate, phenylphosphinic acid, boric acid, phenyl boric acid, trifluoromethane sulfonic acid, para toluene sulfonic acid, phenol, tungstic acid, phosphorus tungstic acid, alkyl carboxylic acid represented by formic acid, acetic acid, trifluoro acetic acid, propionic acid, and butyric acid, aromatic carboxylic acid represented by benzoic acid, phthalic acid, and salicylic acid, and the like. Of these, preferred is an acid containing phosphorus such as phosphoric acid, alkyl phosphoric acid, phenyl phosphoric acid, diphenyl phosphite, and phosphonic acid derivative.

The addition amount of the acid is preferably 0.1 to 100 equivalents and more preferably 1 to 10 equivalents compared to 1 equivalent of the ruthenium compound. By adding the acid in an amount of 0.1 equivalents or more, the reaction rate can be effectively increased. Further, from the viewpoint of the effect of promoting the reaction, it is preferably 100 equivalents or less.

The hydroformylation is preferably carried out in the temperature range of 100°C to 200°C. It is more preferably carried out in the temperature range of 110°C to 180°C. It is particularly preferably carried out in the temperature range of 120°C to 160°C. By carrying out the reaction at the temperature of 100°C or higher, the reaction rate increases so that the reaction can easily progress with high efficiency. Further, by controlling the reaction temperature to 200°C or lower, hydrogenation of the unsaturated bond contained in the tricyclodecene monocarboxylic acid derivative that is represented by the formula (II) can be prevented. When hydrogenation of the unsaturated bond contained in the tricyclodecene monocarboxylic acid derivative that is represented by the formula (II) occurs, the hydroformylation cannot be achieved.

It is necessary to carry out the hydroformylation within a pressure resistant vessel. The reaction is preferably carried out in the reaction pressure range of 1 MPa to 20 MPa. More preferably, it is carried out in the reaction pressure range of 2 MPa to 15 MPa. From the viewpoint of the reaction rate, the pressure is preferably 1 MPa or more. From the viewpoint of the effect of promoting the reaction, it is preferably 20 MPa or less.

The hydroformylation of the invention may be carried out in the presence of a solvent, if necessary. The solvent that can be used is not specifically limited, as long as it can dissolve the tricyclodecene monocarboxylic acid derivative that is represented by the formula (II). Specific examples of the solvent that can be preferably used include n-pentane, n-hexane, n-heptane, cyclohexane, benzene, toluene, o-xylene, p-xylene, m-xylene, ethylbenzene, cumen, tetrahydrofuran, N-methyl pyrrolidone, dimethyl formamide, dimethyl acetamide, dimethyl imidazolidinone, ethylene glycol dimethyl ether, diethylene glycol dimethyl ether, triethylene glycol dimethyl ether, and the like. When the solvent is used, preferred use amount thereof is to yield that concentration of the tricyclodecene monocarboxylic acid derivative that is represented by the formula (II) is within the range of 10 to 1000% by weight.

The tricyclodecane mono-methanol monocarboxylic acid derivative obtained by the manufacturing method of the invention is useful as a raw material for alicyclic polyesters. Further, the alicyclic polyesters in which the derivative is used have excellent heat resistance and transparency, and therefore they can be used for an electronic part used for a semiconductor liquid crystal, optical materials represented by optical fiber, optical lens, and the like, and also a material related to a display and a material related to medical use.

### EXAMPLES

Herein below, the invention is explained in greater detail in view of Examples. However, the scope of the invention is not limited by the following Examples.

Examples 1 to 9 and Comparative Examples 1 to 3: Synthesis of methyl tricyclodecene monocarboxylate

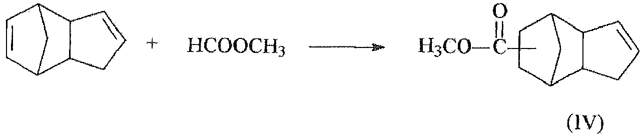

### (Example 1)

At room temperature, 10.0 mmol of dicyclopentadiene and 5.0 ml of methyl formate were added to a catalytic system in which 0.025 mmol of [Ru(CO)₃Cl₂]₂ as a ruthenium compound, 0.025 mmol Co₂(CO)₈ as a cobalt compound, and 0.5 mmol of butyl methyl pyrrolidinium chloride as a halide salt are mixed in a high-pressure reaction apparatus made of stainless steel with inside volume of 50 ml. Subsequently, the reaction apparatus was purged with 0.5 MPa nitrogen gas and maintained at 120°C for 8 hours. After that, the inside of the reaction apparatus was cooled to room temperature and depressurized. Part of the remaining organic phase was extracted and analyzed by gas chromatography. According to the results of analysis, 0.95 mmol (yield: 9.5% based on the dicyclopentadiene) of methyl tricyclodecene monocarboxylate represented by the above formula (IV) was produced. As a result of analysis using gas chromatography-mass analysis (GC-MS), it was found that the methyl tricyclodecene monocarboxylate obtained is a mixture of 8-methoxycarbonyl-tricyclo[5.2.1.0^{2,6}]dec-3-ene and 9-methoxycarbonyl-tricyclo[5.2.1.0^{2,6}]dec-3-ene. The methyl tricyclodecene monocarboxylate obtained was isolated by distillation under reduced pressure.

### (Example 2)

The reaction was carried out all in the same manner as Example 1 except that 2.0 mmol of N-methylpyrrolidine was added as a basic compound to the catalytic system used in Example 1. As a result, it was found that 8.21 mmol (yield: 82.1% based on the dicyclopentadiene) of methyl tricyclodecene monocarboxylate is produced. The methyl tricyclodecene monocarboxylate obtained was isolated by distillation under reduced pressure.

### (Example 3)

The reaction was carried out all in the same manner as Example 1 except that 0.5 mmol of sodium t-butoxide was added as a basic compound to the catalytic system used in ExampleExample 1. As a result, it was found that 8.40 mmol (yield: 84.0% based on the dicyclopentadiene) of methyl tricyclodecene monocarboxylate is produced. The methyl tricyclodecene monocarboxylate obtained was isolated by distillation under reduced pressure.

### (Example 4)

The reaction was carried out all in the same manner as ExampleExample 2 except that 0.5 mmol of p-cresol was added as a phenol compound to the catalytic system used in ExampleExample 2. As a result, it was found that 9.05 mmol (yield: 90.5% based on the dicyclopentadiene) of methyl tricyclodecene monocarboxylate is produced. The methyl tricyclodecene monocarboxylate obtained was isolated by distillation under reduced pressure.

### (Example 5)

The reaction was carried out all in the same manner as ExampleExample 2 except that 0.5 mmol of methylene chloride was added as an organic halogen compound to the catalytic system used in ExampleExample 2. As a result, it was found that 8.69 mmol (yield: 86.9% based on the dicyclopentadiene) of methyl tricyclodecene monocarboxylate is produced. The methyl tricyclodecene monocarboxylate obtained was isolated by distillation under reduced pressure.

### (Example 6)

The reaction was carried out all in the same manner as ExampleExample 1 except that the halide salt was replaced with 0.5 mmol of trioctyl methyl ammonium chloride and 2.0 mmol of triethylamine was added as a basic compound to the catalytic system used in ExampleExample 1. As a result, it was found that 9.23 mmol (yield: 92.3% based on the dicyclopentadiene) of methyl tricyclodecene monocarboxylate is produced. The methyl tricyclodecene monocarboxylate obtained was isolated by distillation under reduced pressure.

### (Example 7)

The reaction was carried out all in the same manner as ExampleExample 6 except that, in the catalytic system used in ExampleExample 6, the ruthenium compound was replaced with the ruthenium compound [Ru(CO)₂Cl₂]ₙ (0.05 mmol based on Ru metal), which has been prepared in advance with RuCl₃ and formic acid according to the method described in M. J. Cleare and W. P. Griffith (J. Chem. Soc. (A), 1969, 372). As a result, it was found that 8.67 mmol (yield: 86.7% based on the dicyclopentadiene) of methyl tricyclodecene monocarboxylate is produced. The methyl tricyclodecene monocarboxylate obtained was isolated by distillation under reduced pressure.

### (Example 8)

The reaction was carried out all in the same manner as Example 6 except that, in the catalytic system used in Example 6, the cobalt compound was replaced with 0.025 mmol cobalt citrate dihydrate. As a result, it was found that 8.87 mmol (yield: 88.7% based on the dicyclopentadiene) of methyl tricyclodecene monocarboxylate is produced. The methyl tricyclodecene monocarboxylate obtained was isolated by distillation under reduced pressure.

### (Example 9)

The reaction was carried out all in the same manner as Example 6 except that, in the catalytic system used in Example 6, the cobalt compound was replaced with 0.05 mmol cobalt acetate tetrahydrate. As a result, it was found that 8.39 mmol (yield: 83.9% based on the dicyclopentadiene) of methyl tricyclodecene monocarboxylate is produced. The methyl tricyclodecene monocarboxylate obtained was isolated by distillation under reduced pressure.

### (Comparative Example 1)

The reaction was carried out all in the same manner as Example 1 except that no cobalt compound is used in the catalytic system of Example 1. As a result, only a trace amount of methyl tricyclodecene monocarboxylate was produced.

### (Comparative Example 2)

The reaction was carried out all in the same manner as Example 1 except that no ruthenium compound is used in the catalytic system of Example 1. As a result, only a trace amount of methyl tricyclodecene monocarboxylate was produced.

### (Comparative Example 3)

The reaction was carried out all in the same manner as Example 1 except that no halide salt is used in the catalytic system of Example 1. As a result, only a trace amount of methyl tricyclodecene monocarboxylate was produced.
The catalyst composition and the yield for Examples 1 to 9 and Comparative Examples 1 to 3 are shown in the following Table 1.

**[Table 1]**

| | | | Example | | | | | | | | | Comparative Example | | |
|---|---|---|---|---|---|---|---|---|---|---|---|---|---|---|
| | | | 1 | 2 | 3 | 4 | 5 | 6 | 7 | 8 | 9 | 1 | 2 | 3 |
| Catalyst composition (mmol) | Ruthenium compound | [Ru(CO)₃Cl₂]₂ | 0.025 | 0.025 | 0.025 | 0.025 | 0.025 | 0.025 | 0 | 0.025 | 0.025 | 0.025 | 0 | 0.025 |
| | | [Ru(CO)₂Cl₂]ₙ | 0 | 0 | 0 | 0 | 0 | 0 | 0.05 | 0 | 0 | 0 | 0 | 0 |
| | Cobalt compound | Co₂(CO)₈ | 0.025 | 0.025 | 0.025 | 0.025 | 0.025 | 0.025 | 0.025 | 0 | 0 | 0 | 0.025 | 0.025 |
| | | Cobalt citrate dihydrate | 0 | 0 | 0 | 0 | 0 | 0 | 0 | 0.025 | 0 | 0 | 0 | 0 |
| | | Cobalt acetate tetrahydrate | 0 | 0 | 0 | 0 | 0 | 0 | 0 | 0 | 0.05 | 0 | 0 | 0 |
| | Halide salt | Butyl methyl pyrrolidinium chloride | 0.5 | 0.5 | 0.5 | 0.5 | 0.5 | 0 | 0 | 0 | 0 | 0.5 | 0.5 | 0 |
| | | Trioctyl methyl ammonium chloride | 0 | 0 | 0 | 0 | 0 | 0.5 | 0.5 | 0.5 | 0.5 | 0 | 0 | 0 |
| | Basic compound | N-Methylpyrrolidine | 0 | 2.0 | 0 | 2.0 | 2.0 | 0 | 0 | 0 | 0 | 0 | 0 | 0 |
| | | Sodium t-butoxide | 0 | 0 | 0.5 | 0 | 0 | 0 | 0 | 0 | 0 | 0 | 0 | 0 |
| | | Triethylamine | 0 | 0 | 0 | 0 | 0 | 2.0 | 2.0 | 2.0 | 2.0 | 0 | 0 | 0 |
| | Phenol compound | p-Cresol | 0 | 0 | 0 | 0.5 | 0 | 0 | 0 | 0 | 0 | 0 | 0 | 0 |
| | Organic halide | Methylene chloride | 0 | 0 | 0 | 0 | 0.5 | 0 | 0 | 0 | 0 | 0 | 0 | 0 |
| Yield (%) | | | 9.5 | 82.1 | 84.0 | 90.5 | 86.9 | 92.3 | 86.7 | 88.7 | 83.9 | trace | trace | trace |

Examples 10 to 14: Synthesis of methyl tricyclodecane mono-methanol monocarboxylate

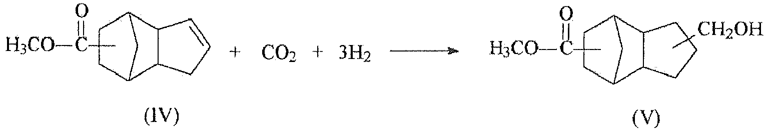

### (Example 10)

At room temperature, 10.0 mmol of methyl tricyclodecene monocarboxylate and 10.0 ml of toluene as solvent were added and stirred to dissolve in a catalytic system in which 0.05 mmol of Ru₂(CO)₆Cl₄ as a ruthenium compound, 2.5 mmol of hexadecyl trimethyl ammonium chloride as a halide salt, and 0.25 mmol of diphenyl phosphite as an acid are mixed, in a high-pressure reaction apparatus made of stainless steel with inside volume of 50 ml. After stirring to dissolve, inside of the reaction system was pressurized with 4 MPa carbon dioxide and 4 MPa hydrogen and maintained at 140°C for 15 hours. After that, the inside of the reaction apparatus was cooled to room temperature and depressurized. Part of the remaining organic phase was extracted and analyzed by gas chromatography. According to the results of analysis, 4.65 mmol (yield: 46.5% based on the methyl tricyclodecene monocarboxylate) of methyl tricyclodecane mono-methanol monocarboxylate represented by the above formula (V) was produced. As a result of analysis using gas chromatography-mass analysis (GC-MS), it was found that the methyl tricyclodecane mono-methanol monocarboxylate obtained is a mixture of 4-hydroxymethyl-8-methoxycarbonyl-tricyclo[5.2.1.0^{2,6}]decane, 3-hydroxymethyl-8-methoxycarbonyl-tricyclo[5.2.1.0^{2,6}]decane, and 3-hydroxymethyl-9-methoxycarbonyl-tricyclo[5.2.1.0^{2,6}] decane.

### (Example 11)

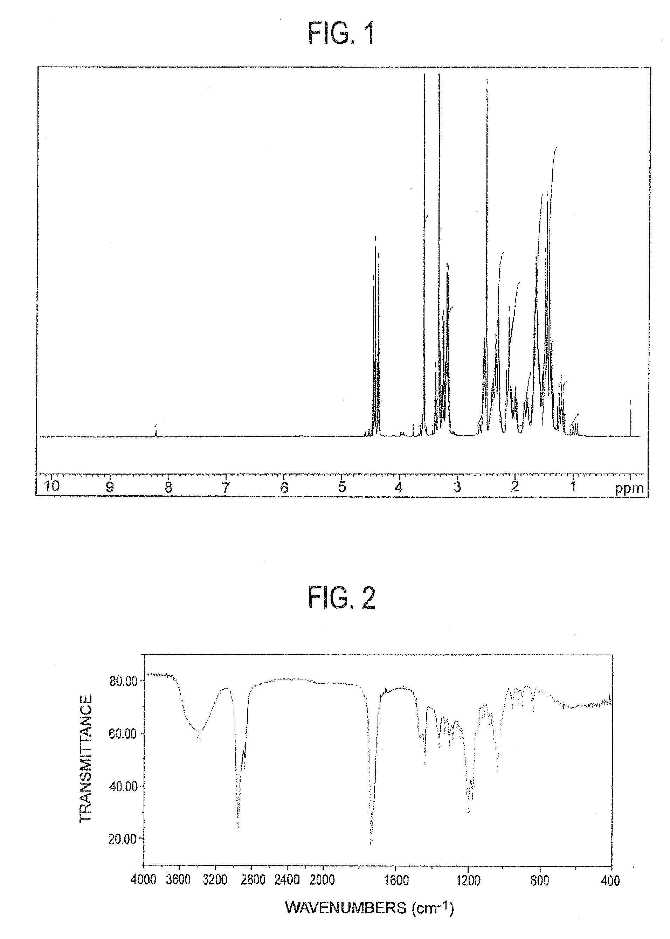

The experiment was carried out in the same manner as Example 10 except that 0.05 mmol of Co₂(CO)₈ was additionally added as a cobalt compound, followed by analysis by gas chromatography. According to the results of analysis, 7.45 mmol (yield: 74.5% based on the methyl tricyclodecene monocarboxylate) of methyl tricyclodecane mono-methanol monocarboxylate represented by the above formula (V) was produced. As a result of analysis using gas chromatography-mass analysis (GC-MS), it was found that the methyl tricyclodecane mono-methanol monocarboxylate obtained is a mixture of 4-hydroxymethyl-8-methoxycarbonyl-tricyclo[5.2.1.0^{2,6}]decane, 3-hydroxymethyl-8-methoxycarbonyl-tricyclo[5.2.1.0^{2,6}]decane, and 3-hydroxymethyl-9-methoxycarbonyl-tricyclo[5.2.1.0^{2,6}]decane.
Methyl tricyclodecane mono-methanol monocarboxylate obtained was isolated by distillation under reduced pressure and subjected to measurement of ¹H-NMR spectrum and IR spectrum. For measurement of ¹H-NMR spectrum, the sample was dissolved in dimethyl sulfoxide (DMSO-d6) to give a solution, which was then added to a sample tube of φ 5 mm and measured by 400 MHz nuclear magnetic resonance machine "AV400M" (trade name, manufactured by Bruker). Further, the IR spectrum was measured by using a Fourier-transformed infrared spectrophotometer (trade name: JIR-6500, manufactured by JEOL Ltd.).
The ¹H-NMR spectrum obtained is given in FIG. 1. Each proton was assigned as described below. From the viewpoint of easy interpretation, 4-hydroxymethyl-8-methoxycarbonyl-tricyclo[5.2.1.0^{2,6}]decane that is expressed as the following formula, was taken as an example.
As a result of the ¹H-NMR analysis (FIG. 1), each proton was assigned as follows. 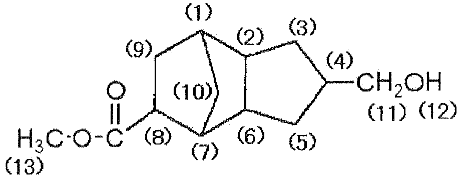
Proton (1): peak near 2.1 ppm
Proton (2): peak near 2.4 ppm
Proton (3): peaks near 1.2 ppm and 1.7 ppm
Proton (4): peak near 2.3 ppm
Proton (5): peaks near 1.2 ppm and 1.8 ppm
Proton (6): peak near 2.6 ppm
Proton (7): peak near 2.1 ppm
Proton (8): peak near 1.9 ppm
Proton (9): peaks near 0.9 ppm and 1.7 ppm
Proton (10): peaks near 1.4 to 1.5 ppm
Proton (11): peak near 3.2 ppm
Proton (12): peak near 4.4 ppm
Proton (13): peak near 3.6 ppm

Integration strength ratio among the Proton (1) to (10) of the tricyclodecane moiety/Proton (11) of the hydroxymethyl group / Proton (12) of the hydroxymethyl group/ Proton (13) of the methoxycarbonyl group was found to be 13.93/2.00/1.04/3.08 (theoretical value: 14/2/1/3), and it was confirmed that the methyl tricyclodecane mono-methanol monocarboxylate obtained has the structure represented by the above formula (V).

Further, the IR spectrum is shown in FIG. 2. Peaks of the methylene group and methine group in the tricyclodecane moiety are found near 800 to 1450 cm⁻¹, the peak of the methylene group originating from the hydroxy methyl group is found near 1465 cm⁻¹, the peak of the hydroxy group originating from the hydroxy methyl group is found near 3400 cm⁻¹ (broad), the peak of the carbonyl group originating from the methoxycarbonyl group is found near 1760 cm⁻¹, and the peak of the methyl group originating from the methoxycarbonyl group is found near 2870 cm⁻¹ and 2960 cm⁻¹.

### (Example 12)

At room temperature, 10.0 mmol of the methyl tricyclodecene monocarboxylate isolated in the above and 10.0 ml of tetrahydrofuran as a solvent were added and stirred to dissolve in a catalytic system in which 0.1 mmol of Ru₂(CO)₆Cl₄ as a ruthenium compound, 0. 1 mmol of Co₂(CO)₈ as a cobalt compound, 2.0 mmol of butyl methyl pyrrolidium chloride as a halide salt, and 0.5 mmol of 4-methoxyphenyl phosphonic acid as an acid are mixed in a high-pressure reaction apparatus made of stainless steel with inside volume of 50 ml. After stirring to dissolve, the reaction system was pressurized with 2 MPa carbon dioxide and 6 MPa hydrogen and maintained at 140°C for 15 hours. After that, the inside of the reaction apparatus was cooled to room temperature and depressurized. Part of the remaining organic phase was extracted and analyzed by gas chromatography. According to the results of analysis, 5.54 mmol (yield: 55.4% based on the methyl tricyclodecene monocarboxylate) of methyl tricyclodecane mono-methanol monocarboxylate represented by the above formula (V) was produced.

### (Example 13)

The reaction was carried out all in the same manner as Example 12 except that the halide salt was replaced with 2.0 mmol of octyl methyl pyrrolidium chloride, followed by analysis by gas chromatography. According to the results of analysis, 6.00 mmol (yield: 60.0% based on the methyl tricyclodecene monocarboxylate) of methyl tricyclodecane mono-methanol monocarboxylate represented by the above formula (V) was produced.

### (Example 14)

The reaction was carried out all in the same manner as Example 13 except that 0.5 mmol of p-cresol was further added as a phenol compound, followed by analysis by gas chromatography. According to the results of analysis, 8.96 mmol (yield: 89.6% based on the methyl tricyclodecene monocarboxylate) of methyl tricyclodecane mono-methanol monocarboxylate represented by the above formula (V) was produced.
The catalyst composition and the yield for Examples 10 to 14 are shown in the following Table 2.

**[Table 2]**

| | | | Example | | | | |
|---|---|---|---|---|---|---|---|
| | | | 10 | 11 | 12 | 13 | 14 |
| Catalyst composition (mmol) | Ruthenium compound | Ru₂(CO)₆Cl₄ | 0.05 | 0.05 | 0.1 | 0.1 | 0.1 |
| | Cobalt compound | Co₂(CO)₈ | 0 | 0.05 | 0.1 | 0.1 | 0.1 |
| | Halide salt | Hexadecyl trimethyl ammonium chloride | 2.5 | 2.5 | 0 | 0 | 0 |
| | | Butyl methyl pyrrolidium chloride | 0 | 0 | 2.0 | 0 | 0 |
| | | Octyl methyl pyrrolidium chloride | 0 | 0 | 0 | 2.0 | 2.0 |
| | Acid | Diphenyl phosphorus acid | 0.25 | 0.25 | 0 | 0 | 0 |
| | | 4-Methoxyphenyl phosphonic acid | 0 | 0 | 0.5 | 0.5 | 0.5 |
| | Phenol compound | p-Cresol | 0 | 0 | 0 | 0 | 0.5 |
| Yield (%) | | | 46.5 | 74.5 | 55.4 | 60.0 | 89.6 |

### (Reference example) [Synthesis of polyesters having tricyclodecane skeleton]

To a 10 ml flask equipped with a stirrer, a nitrogen inlet tube, and a condenser, 5 g of methyl tricyclodecane mono-methanol monocarboxylate obtained from Example 14 and 0.5 g of titanium tetra isopropoxide were added, and stirred for 6 hours in an oil bath at 130°C. As a result, polyesters having tricyclodecane skeleton with number average molecular weight of 30,000 were obtained.

Glass transition temperature (Tg) and initial thermal decomposition temperature (temperature at 5% weight loss, Td₅) were measured according to the following condition for the polyesters having tricyclodecane skeleton.
Results are given in the Table 1.

### (1) Glass transition temperature (Tg)

Differential scanning calorimetry (trade name: DSC Type 8230, manufactured by Rigaku Corporation).

Temperature increase rate: 5°C/min

Atmosphere: air

### (2) Initial thermal decomposition temperature (temperature at 5% weight loss, Td₅)

Differential thermal analyzer (trade name: TG-DTA Type 5200, manufactured by Seiko Instruments Inc.).

Temperature increase rate: 5°C/min

Atmosphere: air

Further, light transmission ratio at each wavelength was measured for the polyesters having tricyclodecane skeleton obtained by using Type V-570 (trade name, manufactured by JASCO Corporation) UV/VIS spectrophotometer. The evaluation results are summarized and given in the Table 3.

**[Table 3]**

| Item | | | Reference example |
|---|---|---|---|
| Polymer characteristics | Glass transition temperature (°C) | | 160 |
| | Initial thermal decomposition temperature (°C) | | 417 |
| | Light transmission ratio (%) | 400nm | 100 |
| | | 500nm | 100 |
| | | 600nm | 100 |

As shown in the Table 3, it was found that the polyesters using the tricyclodecane mono-methanol monocarboxylic acid derivative of the invention had high glass transition temperature of 160°C as well as high initial thermal decomposition temperature of 417°C. Thus, it was shown that the polyesters obtained had extremely excellent heat resistance. Further, as the light transmission ratio of the polyesters was 100% for any wavelength, it was also found that they had sufficient light transmission ratio.

## Claims

1. A method for manufacturing a tricyclodecane mono-methanol monocarboxylic acid derivative, comprising the steps of:
reacting a dicyclopentadiene represented by formula (I) with a formic acid compound in the presence of a catalytic system containing a ruthenium compound, a cobalt compound, and a halide salt, to give a tricyclodecene monocarboxylic acid derivative represented by formula (II); and
hydroformylating the tricyclodecene monocarboxylic acid derivative to give a tricyclodecane mono-methanol monocarboxylic acid derivative represented by formula (III). 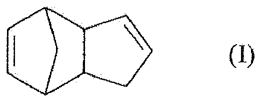 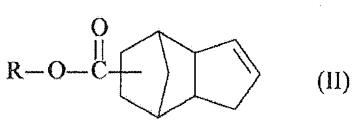 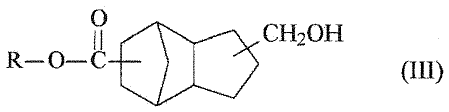 (in the formulae (II) and (III), R represents a hydrogen, an alkyl group having 1 to 5 carbon atoms, a vinyl group, or a benzyl group.)

2. The method for manufacturing a tricyclodecane mono-methanol monocarboxylic acid derivative according to claim 1, wherein the ruthenium compound is a ruthenium complex having both a carbonyl ligand and a halogen ligand in the molecule.

3. The method for manufacturing a tricyclodecane mono-methanol monocarboxylic acid derivative according to claim 1 or 2, wherein the halide salt is a quaternary ammonium salt.

4. The method for manufacturing a tricyclodecane mono-methanol monocarboxylic acid derivative according to any one of claims 1 to 3, wherein the reaction between the dicyclopentadiene and the formic acid compound is performed in the presence of a basic compound.

5. The method for manufacturing a tricyclodecane mono-methanol monocarboxylic acid derivative according to claim 4, wherein the basic compound is a tertiary amine.

6. The method for manufacturing a tricyclodecane mono-methanol monocarboxylic acid derivative according to any one of claims 1 to 5, wherein the reaction between the dicyclopentadiene and the formic acid compound is performed in the presence of a phenol compound.

7. The method for manufacturing a tricyclodecane mono-methanol monocarboxylic acid derivative according to any one of claims 1 to 6, wherein the reaction between the dicyclopentadiene and the formic acid compound is performed in the presence of an organic halide.

8. The method for manufacturing a tricyclodecane mono-methanol monocarboxylic acid derivative according to any one of claims 1 to 7, wherein the hydroformylation of the tricyclodecene monocarboxylic acid derivative is performed in the presence of a catalytic system containing a ruthenium compound.

9. The method for manufacturing a tricyclodecane mono-methanol monocarboxylic acid derivative according to any one of claims 1 to 8, wherein a halide salt is used in combination in the catalytic system for the hydroformylation of the tricyclodecene monocarboxylic acid derivative.

10. The method for manufacturing a tricyclodecane mono-methanol monocarboxylic acid derivative according to any one of claims 1 to 9, wherein a cobalt compound is used in combination in the catalytic system for the hydroformylation of the tricyclodecene monocarboxylic acid derivative.

11. The method for manufacturing a tricyclodecane mono-methanol monocarboxylic acid derivative according to any one of claims 1 to 10, wherein an acid is used in combination in the catalytic system for the hydroformylation of the tricyclodecene monocarboxylic acid derivative.

12. The method for manufacturing a tricyclodecane mono-methanol monocarboxylic acid derivative according to claim 11, wherein the acid is Broensted acid.

13. The method for manufacturing a tricyclodecane mono-methanol monocarboxylic acid derivative according to claim 12, wherein the Broensted acid is an acid containing phosphorus.

14. The method for manufacturing a tricyclodecane mono-methanol monocarboxylic acid derivative according to any one of claims 1 to 13, wherein the hydroformylation of the tricyclodecene monocarboxylic acid derivative is performed in the presence of a phenol compound.

15. The method for manufacturing a tricyclodecane mono-methanol monocarboxylic acid derivative according to any one of claims 1 to 14, wherein the hydroformylation of the tricyclodecene monocarboxylic acid derivative is performed at a reaction temperature of 100 to 200°C and a pressure of 1 to 20 MPa.
